# EUROPÄISCHE PATENTSCHRIFT

(11) **EP 0 576 464 B1**
(45) Veröffentlichungstag und Bekanntmachung des Hinweises auf die Patenterteilung: **21.12.1994**
(21) Anmeldenummer: 92906022.6
(22) Anmeldetag: 12.03.1992
(51) Int. Cl.: C11D 1/14, C07C 303/24

(54) **VERFAHREN ZUR HERSTELLUNG VON HOCHKONZENTRIERTEN FETTALKOHOLSULFAT-PASTEN**
PROCESS FOR PRODUCING HIGHLY CONCENTRATED FATTY ALCOHOL SULPHATE PASTES
PROCEDE DE PREPARATION DE PATES TRES CONCENTREES EN SULFATE D'ALCCOOLS GRAS

(30) Priorität: 21.03.1991 DE 4109250
(43) Veröffentlichungstag der Anmeldung: 05.01.1994
(73) Patentinhaber: Henkel Kommanditgesellschaft auf Aktien, 40191 Düsseldorf (DE)
(72) Erfinder: GRUBER, Bert, D-5012 Bedburg (DE); ANZINGER, Hermann, D-4000 Düsseldorf 13 (DE); HENDRICKS, Günter, D-4000 Düsseldorf 13 (DE)
(86) Internationale Anmeldenummer: EP9200544
(87) Internationale Veröffentlichungsnummer: WO9216606

(56) Entgegenhaltungen:
- EP-A- 0 401 642
- WO-A-91/02045
- US-A- 2 214 254
- US-A- 3 415 753
- US-A- 4 191 704
- THE JOURNAL OF THE AMERICAN OIL CHEMISTS SOCIETY Bd.36,31 Mai 1959,CHAMPAIGN ILLINOIS USA Seiten 208-210; E.E. GILBERT et al.: "SULFATION WITH SULFUR TRIOXIDE: ETHENOXYLATED LONG-CHAIN ALCOHOLS"

## Beschreibung

Die Erfindung betrifft ein Verfahren zur Herstellung von hochkonzentrierten Fettalkoholsulfat-Pasten durch Gassulfierung von Fettalkoholgemischen und anschließende einstufige Neutralisation und Hydrolyse.

Anionische Tenside vom Typ der Fettalkoholsulfate, insbesondere solche, die im Fettrest 16 bis 18 Kohlenstoffatome enthalten, zeigen ausgezeichnete Detergenseigenschaften und eignen sich zur Herstellung von Wasch-, Spül- und Reinigungsmitteln.

Zur Herstellung von Fettalkoholsulfaten geht man von Fettalkoholen aus, die zunächst mit geeigneten Sulfiermitteln, beispielsweise gasförmigem Schwefeltrioxid in die entsprechenden Schwefelsäurehalbester überführt und anschließend mit Basen neutralisiert werden [**J.AM.Oil.Chem.Soc. 36, 208 (1960)**]. Werden als Ausgangsstoffe ungesättigte Fettalkohole eingesetzt, müssen die Reaktionsprodukte zusätzlich zur Neutralisation noch einer Hydrolyse unterworfen werden, um ein Nachsäuern zu verhindern. Entsprechende Verfahren sind beispielsweise in **Ten.Surf.Det 15, 299 (1978)** und **Ind.Eng**. **Chem.Prod.Res.Develop**., **1, 24 (1965)** beschrieben.

Der technische Einsatz von Fettalkoholsulfaten, insbesondere zur Herstellung von pulverförmigen Waschmitteln, ist bislang begrenzt, da schon die Lagerung und der Transport von wäßrigen Fettalkoholsulfat-Pasten mit schwerwiegenden Problemen verbunden ist. So weisen Fettalkoholsulfat-Pasten mit Feststoffgehalten von 30 bis 70 Gew.-% bei Umgebungstemperatur eine so hohe Viskosität und eine so starke Fließanomalie (Rheopexie) auf, daß ein Umfüllen oder Umpumpen praktisch unmöglich ist.

Als Ausweg könnte sich anbieten, die Pasten auf eine ausreichend hohe Temperatur zu erhitzen und auf diesem Wege fließfähig zu machen. Da sich Fettalkoholsulfate jedoch schon bei Temperaturen um 80°C zu Zersetzen beginnen, ist eine Lagerung und Handhabung unter diesen Bedingungen nur bei sorgfältigster pH-Kontrolle und gegebenenfalls Zusatz von Alkali möglich.

Auch die Alternative, die Viskosität von Fettalkoholsulfat-Pasten durch Verdünnen mit Wasser soweit herabzusetzen, daß ein Umpumpen möglich wird, ist unvorteilhaft, da ein überflüssiger Massentransport bei der Sprühtrocknung der Pasten zur Herstellung von pulverförmigen Produkten mit einem zu hohen Energieaufwand verbunden wäre.

In der Vergangenheit ist das Problem der hohen Viskosität wäßriger Aniontensid-Pasten Gegenstand einer Vielzahl von Untersuchungen gewesen. Stellvertretend soll an dieser Stelle auf die Patentanmeldungen **EP 0 24 711 A1**, **EP 0 116 905 A1** und **DE 16 17 160** verwiesen werden, in denen die Verwendung von alkoxylierten Alkoholen, Cumolsulfonat oder Phosphorsäureestern als Viskositätsminderer für Fettalkoholsulfat-Pasten vorgeschlagen wird. Derartige Verfahren sind jedoch grundsätzlich mit dem Nachteil behaftet, daß das Zumischen von Additiven mit zusätzlichem technischen Aufwand verbunden ist und zudem die anwendungstechnischen Eigenschaften der Produkte nachteilig beeinflußen kann.

Die Aufgabe der Erfindung bestand somit darin, ein Verfahren zur Herstellung von hochkonzentrierten Fettalkoholsulfat-Pasten bereitzustellen, das frei von den geschilderten Nachteilen ist.

Gegenstand der Erfindung ist ein Verfahren zur Herstellung von hochkonzentrierten Fettalkoholsulfat-Pasten durch Umsetzung von Fettalkoholen mit gasförmigem Schwefeltrioxid und anschließende Neutralisation und Hydrolyse, das sich dadurch auszeichnet, daß man Gemische enthaltend
a) ungesättigte Fettalkohole der Formel **(I)**,

   **R¹-OH** **(I)**

   in der R¹ für einen linearen ungesättigten Kohlenwasserstoffrest mit 16 bis 22 Kohlenstoffatomen und 1, 2 oder 3 Doppelbindungen steht und
b) gesättigte Fettalkohole der Formel **(II)**,

   **R²-OH (II)**

   in der R² für einen linearen oder verzweigten gesättigten Kohlenwasserstoffrest mit 12 bis 22 Kohlenstoffatomen steht
gemeinsam mit einem Schwefeltrioxid/Inertgas-Gemisch in einem molaren Verhältnis von 1 : 0,95 bis 1 : 1,3 (Mole Fettalkohol pro Mole Schwefeltrioxid) umsetzt und die Reaktionsprodukte bei Temperaturen von 50 bis 90°C in einem Schritt mit einer derartigen Menge wäßriger Base neutralisiert und hydrolysiert, daß sich wäßrige Fettalkoholsulfat-Pasten mit einem Feststoffgehalt von 60 bis 75 Gew.-% - bezogen auf die Paste - ergeben, die bei einer Temperatur von 60°C eine Viskosität von weniger als 10 Pa·s aufweisen.

Überraschenderweise wurde gefunden, daß Abmischungen von ungesättigten und gesättigten Fettalkoholen nach Sulfierung, Neutralisation und Hydrolyse eine geringe Viskosität und eine niedrige Fließgrenze aufweisen, so daß das für Fettalkoholsulfate auf Basis ausschließlich gesättigter Fettalkohole typische rheopexe Fließverhalten überwunden wird. Hierdurch ist es möglich, Fettalkoholsulfat-Pasten mit hohem Feststoffgehalt herzustellen, die auch bei niedrigen Temperaturen und geringem Schergefälle fließ- und pumpfähig sind.

Mit der Erfindung wird ferner das bisher geltende Vorurteil überwunden, nach dem die Neutralisation und die Hydrolyse von sauren Sulfierprodukten auf der Basis ungesättigter Ausgangsstoffe stets in zwei Stufen und mit einem großen Überschuß an Base durchgeführt werden muß. Es wurde vielmehr gefunden, daß sich diese beiden Reaktionen in einem Schritt und mit - auf die Menge des eingesetzten Sulfieragens bezogen - äquimolaren Mengen Base durchführen lassen, sofern man bei ausreichend hohen Temperaturen, beispielsweise 70 bis 90°C arbeitet.

Als typische Beispiele für ungesättigte Fettalkohole der Formel **(I)**, die im Sinne der Erfindung als Komponente a) dienen, kommen Palmitoleylalkohol, Oleylalkohol, Elaidylalkohol, Petroselinylalkohol, Linolylalkohol, Linolenylalkohol, Gadoleylalkohol oder Erucylalkohol in Betracht. Bevorzugt ist der Einsatz von Oleylalkohol.

Unter gesättigten Fettalkoholen der Formel **(II)**, die im Sinne der Erfindung als Komponente b) dienen, sind zum einen die weitgehend linearen Fettalkohole zu verstehen, welche durch Hochdruckhydrierung von Fettsäurealkylestern auf Basis natürlicher Fette und Öle erhalten werden. Typische Beispiele sind Laurylalkohol, Myristylalkohol, Cetylalkohol, Stearylalkohol, Arachylalkohol oder Behenylalkohol. Zum anderen kommen auch die teilweise verzweigten Oxoalkohole des genannten C-Zahlbereiches in Betracht, die durch Hydrierung der entsprechenden Aldehyde aus der Roelen'schen Oxo-Synthese zugänglich sind. Bevorzugt ist der Einsatz von Cetyl- und/oder Stearylalkohol.

Die Mischung der Komponenten a) und b) kann auf rein mechanischem Wege, beispielsweise durch Verrühren bei gegebenenfalls erhöhter Temperatur erfolgen. Hierbei kann das Gewichtsverhältnis der ungesättigten Fettalkohole zu den gesättigten Fettalkoholen 99 : 1 bis 50 : 50, insbesondere 95 : 5 bis 65 : 35 betragen.

Vorzugsweise setzt man jedoch technische Gemische in die Sulfierung ein, die die Komponenten a) und b) direkt in den angegebenen Gewichtsverhältnissen enthalten. Wie dem Fachmann bekannt ist, geht man hierzu üblicherweise von Fetten oder Ölen hoher Iodzahl, beispielsweise Rüböl, Sonnenblumenöl, Erdnußöl, Baumwollsaatöl, Korianderöl, Sojaöl, Leinöl oder Rindertalg aus, die zunächst in die Methylester überführt und anschließend unter Erhalt der Doppelbindungen ihrer ungesättigten Anteile zu den Alkoholen hydriert werden.

Die Sulfierung der Fettalkoholgemische enthaltend die Komponenten a) und b) mit gasförmigem Schwefeltrioxid kann in der für Fettsäure-niedrigalkylester bekannten Weise [**J.Falbe (ed.)**, "**Surfactants in consumer products**", **Springer Verlag, Berlin-Heidelberg, 1987, S. 61**] erfolgen, wobei kontinuierlich arbeitende Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Das Einsatzverhältnis des gasförmigen Schwefeltrioxids beträgt 0,95 bis 1,3 Mol, vorzugsweise jedoch 1,05 bis 1,1 Mol Schwefeltrioxid pro Mol Fettalkohol. Die Sulfierreaktion kann bei Temperaturen T¹ von 25 bis 70°C, insbesondere jedoch 30 bis 50°C durchgeführt werden.

Die bei der Sulfierung anfallenden sauren Sulfierprodukte werden in wäßrigen Basen eingerührt, neutralisiert und gleichzeitig hydrolysiert, um ein Nachsäuern der Verbindungen zu verhindern. Die Neutralisation/Hydrolyse kann bei Temperaturen von 70 bis 90°C durchgeführt werden. Hierbei werden Anteile an gebildeten Sultonen unter Bildung von Sulfonatem hydrolysiert, ohne daß es zu einer Abspaltung der Sulfatgruppe kommt. In einer bevorzugten Ausführungsform des erfindungsgemäßen Verfahrens werden die sauren Sulfierproduktn unmittelbar nach Verlassen des Reaktors ohne weitere Abkühlung neutralisiert, wobei die Hydrolyse gleichzeitig miterfolgt.

Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine in Betracht. Die Neutralisationsbasen gelangen dabei vorzugsweise in Form 20 bis 50 gew. -%iger wäßriger Lösungen zum Einsatz, wobei wäßrige Natriumhydroxidlösungen bevorzugt sind.

Das Sulfierprodukt stellt ein Gemisch dar, das im wesentlichen Alkyl- und Alkenylsulfate enthält. Da bei Einsatz ungesättigter Fettalkohole in die Sulfierung auch eine Anlagerung des Schwefeltrioxids an die Doppelbindung stattfindet, sind in der Mischung auch Stoffe enthalten, die eine innenständige Sulfonatgruppe beziehungsweise eine Sulfonat- und eine Sulfatgruppe enthalten. Der Anteil dieser innenständigen Sulfierprodukte kann üblicherweise 10 bis 25 Gew.-% - bezogen auf die Menge an Aniontensid - betragen. Es ist nicht auszuschließen, daß der unerwartete Effekt des vorteilhaften rheologischen Verhaltens an das Vorhandensein innenständiger Sulfierprodukte in den angegebenen Mengen gebunden ist.

Die Sulfierprodukte können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfierprodukte, 0,2 bis 2 Gew.-% Wasserstoffperoxid, berechnet als 100 %ige Substanz, oder entsprechende Mengen Natriumhypochlorit eingesetzt. Der pH-Wert der Lösungen kann durch Zusatz von Alkali auf Werte von 7,5 bis 10 eingestellt werden. Zur Stabilisierung gegen Bakterienbefall emfiehlt sich ferner eine Konservierung, z. B. mit Formaldehydlöung, p-Hydroxybenzoat, Sorbinsäure oder anderen bekannten Konservieungsstoffen.

Die nach dem erfindungsgemäßen Verfahren erhältlichen hochkonzentrierten Fettalkoholsulfat-Pasten weisen ausgezeichnete Detergenseigenschaften und eine hohe Kaltwasserlöslichkeit auf. Sie eignen sich daher zur Herstellung von pulverförmigen oder flüssigen Wasch-, Spül- und Reinigungsmitteln sowie Produkten der Haar- und Körperpflege, in denen sie in Mengen von 1 bis 25 Gew.-% - bezogen auf den Feststoffgehalt der Mittel - enthalten sein können.

Die folgenden Beispiele sollen den Gegenstand der Erfindung näher erläutern, ohne ihn darauf einzuschränken.

### Beispiele

### I. HERSTELLUNGSBEISPIELE

### Beispiel 1:

In einem 1-l-Sulfierreaktor mit Mantelkühlung und Gaseinleitungsrohr wurden 260 g (1 mol) eines technischen Oleyl-Cetylalkohols (HD-Ocenol^{(R)} 50-55, Verkaufsprodukt der Fa. Henkel KGaA) der folgenden Zusammensetzung

| | |
|---|---|
| Myristylalkohol | 5 Gew.-% |
| Cetylalkohol | 30 Gew.-% |
| Oleylalkohol | 65 Gew.-% |
| Iodzahl | 53 |
| Hydroxylzahl | 215 |

vorgelegt und bei T¹ = 45°C mit 84 g (1,05 mol) Schwefeltrioxid umgesetzt. Das Schwefeltrioxid wurde durch Erhitzen aus einer entsprechenden Menge 65 gew.-%igen Oleums ausgetrieben, mit Stickstoff auf eine Konzentration von 5 Vol.-% verdünnt und innerhalb von 30 min in das Ausgangsprodukt eingeleitet. Das rohe Sulfierprodukt wurde im Anschluß bei einer Temperatur T² von 80°C mit 214 g einer 20 gew.-%igen wäßrigen Natriumhydroxyidlösung neutralisiert und gleichzeitig hydrolysiert. Anschließend wurde das Reaktionsprodukt mit Natronlauge auf einen pH-Wert von 10 eingestellt.

### Kenndaten des Produktes:

| | |
|---|---|
| Aniontensidgehalt | 65,4 Gew.-% |
| - Sulfatanteil | 53,1 Gew.-% |
| - Sulfonatanteil | 12,3 Gew.-% |
| Unsulfierte Anteile | 2,3 Gew.-% |
| Natriumsulfat | 1,4 Gew.-% |
| Wasser | 30,9 Gew.-% |

Der Aniontensidgehalt und die unsulfierten Anteile wurden nach den DGF-Einheitsmethoden, Stuttgart, 1950-1984, H-III-10 bzw. G-II-6b ermittelt. Der Sulfatgehalt wurde als Natriumsulfat berechnet, die Bestimmung des Wassergehaltes erfolgte nach der Fischer-Methode.

### Beispiel 2:

In einem kontinuierlich arbeitenden Fallfilmreaktor (Länge 120 cm, Querschnitt 1 cm, Eduktdurchsatz 600 g/h) mit Mantelkühlung und seitlicher SO₃-Begasung wurden 2600 g (10 mol) eines technischen Oleylalkohols (HD-Ocenol^{(R)} 90-95, Verkaufsprodukt der Fa.Henkel KGaA) der folgenden Zusammensetzung

| | |
|---|---|
| Myristylalkohol | 1 Gew.-% |
| Cetylalkohol | 5 Gew.-% |
| Oleylalkohol | 94 Gew.-% |
| Iodzahl | 93 |
| Hydroxylzahl | 210 |

bei 50°C mit 880 g (11 mol) gasförmigem Schwefeltrioxid zur Reaktion gebracht. Das saure Reaktionsgemisch wurde dabei kontinuierlich bei T² = 80°C in 50 gew.-%ige Natriumhydroxidlösung eingetragen und gleichzeitig neutralisiert und hydrolysiert.

### Kenndaten des Produktes:

| | |
|---|---|
| Aniontensidgehalt | 69,3 Gew.-% |
| - Sulfatanteil | 57,7 Gew.-% |
| - Sulfonatanteil | 11,6 Gew.-% |
| Unsulfierte Anteile | 2,2 Gew.-% |
| Natriumsulfat | 1,2 Gew.-% |
| Wasser | 27,3 Gew.-% |

### Beispiel 3

In einem 4-Rohrreaktor (Länge 12 m, Rohstoffdurchsatz 100 kg/h)) der Fa.Ballestra wurden 4000 kg eines technischen Oleyl-Cetylalkohols (HD-Ocenol® 50/55, Fa.Henkel KGaA) mit einem Schwefeltrioxid/Luft-Gemisch (5 Vol.-% SO₃, Strömungsgeschwindigkeit 179 m³/h) umgesetzt, wobei die Menge des Sulfiermittels so bemessen wurde, daß sich über die gesamte Dauer der Reaktion im sauren Reaktionsprodukt eine Säurezahl von 160 bis 164 einstellte. Der Alkohol wurde mit einer Temperatur von 45°C in den Reaktor eingebracht, die Temperatur des SO₃/Luftgemisches betrug 27°C, die Temperatur des Reaktorkühlwassers 40 bis 45°C. Nach Verlassen des Reaktors wurde das saure Sulfierprodukt, das eine Temperatur von 50°C aufwies, bei T² = 70°C mit einer solchen Menge 50 gew.-%iger Natronlauge neutralisiert und hydrolysiert, daß sich eine Feststoffkonzentration in der Paste von 73,2 Gew.-% ergab.

Das Reaktionsendprodukt wurde auf pH = 10 eingestellt und mit 1 Gew.-% - bezogen auf den Feststoffgehalt - einer 13 gew.-%igen wäßrigen Lösung von Natriumhypochlorit gebleicht.

### Kenndaten des Produktes:

| | |
|---|---|
| Aniontensidgehalt | 66,6 Gew.-% |
| - Sulfatanteil | 52,6 Gew.-% |
| - Sulfonatanteil | 14,0 Gew.-% |
| Unsulfierte Anteile | 3,2 Gew.-% |
| Natriumsulfat | 2,3 Gew.-% |
| Wasser | 27,9 Gew.-% |

### II. BESTIMMUNG VON FLIESSGRENZE UND VISKOSITÄT

### Beispiele 4 bis 8, Vergleichsbeispiel V1

Die rheologischen Messungen wurden mit einem schubspannungskontrollierten Rotationsrheometer Carri-Med CS 100 mit einem Platte-Platte-Meßsystem im Temperaturbereich 20 bis 80°C durchgeführt. Die Ergebnisse sind in Tab.1 und 2 zusammengefaßt.

**Tab.1**

| Fließgrenzen (Angabe in Pa) | | | | |
|---|---|---|---|---|
| Bsp. | Fettalkoholsulfat-Paste | Temperatur (°C) | | |
| | | 20 | 40 | 60 |
| 4 | gemäß Bsp.3 | 105 | 48 | 7 |
| V1 | Sulfopon^{(R)} T | - | - | 63 |

**Tab.2**

| Viskosität (Angabe in Pa·s) | | | | | |
|---|---|---|---|---|---|
| Bsp. | Fettalkoholsulfat-Paste | T °C | Schergefälle | | |
| | | | 10/s | 30/s | 50/s |
| 5 | gemäß Bsp.3 | 20 | 54 | 21 | 14 |
| 6 | gemäß Bsp.3 | 40 | 32 | 14 | 10 |
| 7 | gemäß Bsp.3 | 60 | 6 | 5 | 4 |
| 8 | gemäß Bsp.3 | 80 | 3 | 2 | 1 |

### Sulfopon^{(R)} T :

Fettalkoholsulfat-Paste auf Basis von gesättigtem C_{16/18}-Talgfettalkohol (Verkaufsprodukt Fa.Henkel KGaA). Die Fließgrenzen bei den Temperaturen 20 und 40°C waren für diese Paste nicht mehr bestimmbar. Ferner ist die Paste unterhalb von 60°C nicht mehr fließfähig.

## Patentansprüche

1. Verfahren zur Herstellung von hochkonzentrierten Fettalkoholsulfat-Pasten durch Umsetzung von Fettalkoholen mit gasförmigem Schwefeltrioxid und anschließende Neutralisation und Hydrolyse, **dadurch gekennzeichnet**, daß man Gemische enthaltend
a) ungesättigte Fettalkohole der Formel **(I)**,
**R¹-OH** **(I)**
in der R¹ für einen linearen ungesättigten Kohlenwasserstoffrest mit 16 bis 22 Kohlenstoffatomen und 1, 2 oder 3 Doppelbindungen steht und
b) gesättigte Fettalkohole der Formel **(II)**,
**R²-OH** **(II)**
in der R² für einen linearen oder verzweigten gesättigten Kohlenwasserstoffrest mit 12 bis 22 Kohlenstoffatomen steht,
gemeinsam mit einem Schwefeltrioxid/Inertgas-Gemisch in einem molaren Verhältnis von 1 : 0,95 bis 1 : 1,3 (Mole Fettalkohol pro Mole Schwefeltrioxid) umsetzt und die Reaktionsprodukte bei Temperaturen von 50 bis 90°C in einem Schritt mit einer derartigen Menge wäßriger Base neutralisiert und hydrolysiert, daß sich wäßrige Fettalkoholsulfat-Pasten mit einem Feststoffgehalt von 60 bis 75 Gew.-% - bezogen auf die Paste - ergeben, die bei einer Temperatur von 60°C eine Viskosität von weniger als 10 Pa·s aufweisen.

2. Verfahren nach Anspruch 1, **dadurch gekennzeichnet**, daß man als ungesättigten Fettalkohohol der Formel **(I)** Oleylalkohol einsetzt.

3. Verfahren nach den Ansprüchen 1 und 2, **dadurch gekennzeichnet**, daß man als gesättigten Fettalkohol der Formel **(II)** Cetyl- und/oder Stearylalkohol einsetzt.

4. Verfahren nach mindestens einem der Ansprüche 1 bis 3, **dadurch gekennzeichnet**, daß das Gewichtsverhältnis zwischen den ungesättigten Fettalkoholen der Formel **(I)** und den gesättigten Fettalkoholen der Formel **(II)** 99 : 1 bis 50 : 50 beträgt.

5. Verfahren nach mindestens einem der Ansprüche 1 bis 4, **dadurch gekennzeichnet**, daß man die Sulfierung bei Temperaturen von 25 bis 70°C durchführt.

6. Verfahren nach mindestens einem der Ansprüche 1 bis 5, **dadurch gekennzeichnet**, daß man SO₃/Inertgas-Gemische einsetzt, in denen der Anteil des Schwefeltrioxids 1 bis 8 Vol.-% beträgt.

7. Verfahren nach mindestens einem der Ansprüche 1 bis 6, **dadurch gekennzeichnet**, daß man die Sulfierung in einem kontinuierlich arbeitenden Reaktor durchführt, der nach dem Fallfilm-Prinzip arbeitet.

8. Verfahren nach mindestens einem der Ansprüche 1 bis 7, **dadurch gekennzeichnet**, daß man die Neutralisation/Hydrolyse mit wäßrigen 20 bis 50 gew.-%igen Lösungen von Alkalihydroxiden durchführt.

## Claims

1. A process for the production of highly concentrated fatty alcohol sulfate pastes by reaction of fatty alcohols with gaseous sulfur trioxide and subsequent neutralization and hydrolysis, **characterized in that** mixtures containing
a) unsaturated fatty alcohols corresponding to formula **(I)**
**R¹OH** **(I)**
in which R¹ is a linear unsaturated hydrocarbon radical containing 16 to 22 carbon atoms and 1, 2 or 3 double bonds, and
b) saturated fatty alcohols corresponding to formula **(II)**
**R²-OH** **(II)**
in which R² is a linear or branched saturated hydrocarbon radical containing 12 to 22 carbon atoms,
are reacted together with a sulfur trioxide/inert gas mixture in a molar ratio of 1:0.95 to 1:1.3 (mole fatty alcohol per mole sulfur trioxide) and the reaction products are neutralized in one step carried out at 50 to 90°C with such a quantity of aqueous base that water-containing fatty alcohol sulfate pastes with a solids content of 60 to 70% by weight (based on the paste), which have a viscosity of less than 10 Pa·s at a temperature of 60°C, are obtained.

2. A process as claimed in claim 1, **characterized in that** oleyl alcohol is used as the unsaturated fatty alcohol corresponding to formula **(I)**.

3. A process as claimed in claims 1 and 2, **characterized in that** cetyl and/or stearyl alcohol is used as the saturated fatty alcohol corresponding to formula **(II)**.

4. A process as claimed in at least one of claims 1 to 3, **characterized in that** the ratio by weight between the unsaturated fatty alcohols of formula **(I)** and the saturated fatty alcohols of formula **(II)** is 99:1 to 50:50.

5. A process as claimed in at least one of claims 1 to 4, **characterized in that** the sulfonation is carried out at temperatures of 25 to 70°C.

6. A process as claimed in at least one of claims 1 to 5, **characterized in that** SO₃/inert gas mixtures containing 1 to 8% by volume of sulfur trioxide are used.

7. A process as claimed in at least one of claims 1 to 6, **characterized in that** the sulfonation is carried out in a continuous falling-film reactor.

8. A process as claimed in at least one of claims 1 to 7, **characterized in that** the neutralization/hydrolysis step is carried out with aqueous 20 to 50% by weight solutions of alkali metal hydroxides.

## Revendications

1. Procédé de préparation de pâtes très concentrées en sulfate d'alcools gras par réaction d'alcools gras avec du trioxyde de soufre gazeux et neutralisation et hydrolyse subséquentes, caractérisé en ce que des mélanges contenant
a) des alcools gras insaturés de formule (I)
R¹-OH (I)
dans laquelle R¹ désigne un radical hydrocarbure insaturé linéaire avec 16 à 22 atomes de carbone et 1, 2 ou 3 doubles liaisons et
b) des alcools gras saturés de formule (II)
R²-OH (II)
dans laquelle R² désigne un radical hydrocarbure saturé linéaire ou ramifié avec 12 à 22 atomes de carbone,
sont amenés à réagir avec un mélange trioxyde de soufre/gaz inerte dans un rapport molaire de 1:0,95 à 1:1,3 mole d'alcool gras par mole de trioxyde de soufre et les produits réactifs sont neutralisés et hydrolysés à des températures de 50 à 90°C en une seule étape avec une telle quantité de base aqueuse que des pâtes aqueuses de sulfate d'alcools gras avec une teneur en matières solides de 60 à 75 % en poids par rapport à la pâte sont obtenues et présentent, à une température de 60°C, une viscosité de moins de 10 Pa.s.

2. Procédé selon la revendication 1, caractérisé en ce que l'alcool oléylique est utilisé comme alcool gras insaturé de formule (I).

3. Procédé selon les revendications 1 et 2, caractérisé en ce que l'alcool cétylique et/ou l'alcool stéarylique est utilisé comme alcool gras saturé de formule (II).

4. Procédé selon au moins une des revendications 1 à 3, caractérisé en ce que le rapport pondéral entre les alcools gras insaturés de formule (I) et les alcools gras saturés de formule (II) s'élève à 99:1 - 50:50.

5. Procédé selon au moins une des revendications 1 à 4, caractérisé en ce que la formation de sulfate est réalisée à des températures de 25 à 70°C.

6. Procédé selon au moins une des revendications 1 à 5, caractérisé en ce que des mélanges de SO₃/gaz inerte dans lesquels la part de trioxyde de soufre s'élève de 1 à 8 % en volume sont utilisés.

7. Procédé selon au moins une des revendications 1 à 6, caractérisé en ce que la formation de sulfate se déroule dans un réacteur en continu qui fonctionne selon le principe du film ruisselant.

8. Procédé selon au moins une des revendications 1 à 7, caractérisé en ce que les neutralisation/hydrolyse sont réalisées avec des solutions aqueuses à 20 - 50 % en poids d'hydroxydes alcalins.
